# EUROPÄISCHE PATENTSCHRIFT

(11) **EP 0 572 474 B1**
(45) Veröffentlichungstag und Bekanntmachung des Hinweises auf die Patenterteilung: **19.07.1995**
(21) Anmeldenummer: 92905065.6
(22) Anmeldetag: 22.02.1992
(51) Int. Cl.: A61B 17/12, A61M 5/42

(54) **ABSCHNÜRVORRICHTUNG FÜR KÖRPERTEILE**
CONSTRICTION DEVICE FOR REDUCING THE BLOOD FLOW IN PARTS OF THE BODY
DISPOSITIF DE LIGATURE POUR PARTIES DU CORPS

(30) Priorität: 23.02.1991 DE 4105789; 23.02.1991 DE 9102166 U; 11.05.1991 DE 4115515
(43) Veröffentlichungstag der Anmeldung: 08.12.1993
(73) Patentinhaber: PRÄMETA Gesellschaft für Präzisionsmetall- und Kunststofferzeugnisse mbH & Co. KG, 51107 Köln (DE)
(72) Erfinder: SIMON, Pal, D-52538 Gangelt-Hastenrath (DE); WEHKING, Wolfgang, D-5000 Köln 90 (DE)
(74) Vertreter: Dallmeyer, Georg, Dipl.-Ing.
(86) Internationale Anmeldenummer: EP9200381
(87) Internationale Veröffentlichungsnummer: WO9214410

(56) Entgegenhaltungen:
- DE-C- 366 487
- US-A- 1 824 516
- US-A- 2 234 961
- US-A- 3 760 803
- US-A- 4 314 568
- US-A- 4 834 802

## Beschreibung

Die vorliegende Erfindung betrifft eine Abschnürvorrichtung für Körperteile nach dem Oberbegriff des Anspruchs 1.

Abschnürvorrichtungen dieser Art (EP-A 0 375 901) werden in der Medizin verwendet, um eine Vene des Patienten vor einer Venenpunktion zu stauen. Bei diesen bekannten Abschnürvorrichtungen wird zwar ein Blutstau in der Vene erzeugt, es erfolgt jedoch keine Fixierung der Vene. Insbesondere bei älteren Patienten ist das Phänomen der Rollvene bekannt, bei dem es sich um eine alters bedingte Bindegewebsschwäche handelt, durch die die Venen sich locker im Unterhautgewebe bewegen. Bei solchen Rollvenen ist eine Venenpunktion oft nicht oder nur unter großen Schwierigkeiten möglich.

Der vorliegenden Erfindung liegt daher die Aufgabe zugrunde, eine Abschnürvorrichtung der eingangs genannten Art, die um die Extremität des Patienten gelegt wird, zu schaffen, mittels derer nicht nur eine Stauung im Körperteil, sondern gleichzeitig auch eine Fixierung der Vene möglich ist.

Zur Lösung dieser Aufgabe dienen erfindungsgemaß die Merkmale des Anspruchs 1.

Die Erfindung schafft in vorteilhafter Weise eine Abschnürvorrichtung mit einer Fixiereinrichtung für Venen, die das Problem der Rollvenen löst, indem die Fixiereinrichtung einen Aufnahmekanal für die Venen enthält. Der Aufnahmekanal faßt beim Abschnüren des Körperteils die Vene ein und fixiert diese gegen Weggleiten.

Die Abschnürvorrichtung weist eine Fixiereinrichtung aufweisen, die an einer Stelle mit dem im Umfang einstellbaren Abschnürmittel, das um die Extremität gelegt wird, verbunden ist und die einen Spalt mit einer zur Fixierung einer Vene ausreichenden Breite aufweist. Nach dem Anlegen und Festziehen der Abschnürvorrichtung wird folglich die Fixiereinrichtung an den Arm oder das Bein des Patienten angepreßt, wobei die Abschnürvorrichtung so angebracht wird, daß die zu punktierende Vene in dem Spalt der Fixiereinrichtung liegt und dadurch fixiert wird.

Die Fixiereinrichtung ist an dem Schloßteil befestigt. Auf diese Weise kann die Fixiereinrichtung mit dem Schloßteil positioniert werden, was die Handhabung der Abschnürvorrichtung erleichtert.

Dabei kann die Fixiereinrichtung auf einer Seite das eine Ende des Abschnürmittels aufnehmen und auf der diesem Ende gegenüberliegenden Seite ein Verschlußteil aufweisen, das in dem Schloßteil einrastbar ist. Dadurch ist das Anlegen der Abschnürvorrichtung vereinfacht, da das Abschnürmittel mit der Fixiereinrichtung am Ende des Bandes in einfacher Weise um das abzubindende Körperteil gelegt werden kann, wobei durch das Einrasten der Fixiereinrichtung in dem Schloßteil die Schlinge um das Körperteil geschlossen wird und anschließend die Schlinge gespannt werden kann.

Es ist vorgesehen, daß der Spalt der Fixiereinrichtung auf einer Seite nach außen zum Rand der Fixiereinrichtung hin offen ist, wohingegen der Spalt auf der anderen Seite mit Abstand vom Rand der Fixiereinrichtung endet, so daß dort eine Art Steg der Fixiereinrichtung stehenbleibt, der die beiden rechts und links des Spaltes liegenden Hälften der Fixiereinrichtung verbindet. Dieser Steg ist nach oben hin aufgewölbt, so daß nach dem Anlegen der Abschnürvorrichtung zwischen der Unterseite der Fixiereinrichtung und der Extremität des Patienten unter dem Steg eine Art tunnelartiger Freiraum verbleibt.

Die zu punktierende Vene wird selbst nur fixiert und nicht gestaut, die Venen in der Umgebung dieser Vene werden aber gestaut, so daß die fixierte Vene durch den erhöhten Blutrückfluß verstärkt mit Blut durchflossen wird und dadurch gut punktiert werden kann.

Die Abschnürvorrichtung bzw. die Fixiereinrichtung kann auch mit einem schwenkbaren Aufnahmekanal kombiniert sein. Die Schwenkbarkeit des Aufnahmekanals kann dabei entweder durch eine schwenkbare Fixiereinrichtung erreicht werden oder durch schwenkbar an der Fixiereinrichtung befestigte Enden des Abschnürmittels, oder durch einen innerhalb der Fixiereinrichtung schwenkbaren Aufnahmekanal.

Die Fixiereinrichtung kann zumindest in Teilbereichen ihres Umfangs im Umriß kreisrund ausgebildet sein, wobei vorzugsweise die Befestigungspunkte des um die Extremität legbaren Abschnürmittel am Umfang der Fixiereinrichtung entlang einer Kreisbahn zumindest über einen gewissen Kreisbogenbereich verschiebbar sind. Dadurch ist es möglich, die Fixiereinrichtung gegenüber dem Abschnürmittel zu verdrehen und so in eine Position zu bringen, in der die Vene in dem Spalt der Fixiereinrichtung verläuft.

Weiter ist es vorzugsweise vorgesehen, daß der Spalt der Fixiereinrichtung in seiner Breite einstellbar ist. Dies kann technisch zum Beispiel so gelöst werden, daß man die Fixiereinrichtung aus zwei miteinander verbundenen und gegeneinander verschiebbaren Teilplatten herstellt, so daß sich die Spaltbreite des Aufnahmekanals verändert. Dies ermöglicht es, die Spaltbreite der Breite der Vene anzupassen.

Es bietet sich weiterhin an, das eine Ende des Abschnürmittels, vorzugsweise ein Band an der Fixiereinrichtung jeweils lösbar zu befestigen, so daß das Band bei Verschmutzungen ausgewechselt werden kann. Außerdem kann die Fixiereinrichtung an ihrer Unterseite etwas gewölbt sein und zwar entsprechend der Form der Gliedmaße, an die die Abschnürvorrichtung angelegt wird, wodurch das Anlegen der Fixiereinrichtung an dem jeweiligen Körperteil des Patienten verbessert wird. Unterschiedliche Fixiereinrichtungn zum Auswechseln für bestimmte Anwendungsfälle können vorteilhaft vorgesehen sein.

Im folgenden werden Ausführungsbeispiele der Erfindung unter Bezugnahme auf die Zeichnungen näher erläutert.

Es zeigen:
- Fig. 1: eine mit einem Schloßteil gekoppelte Fixiereinrichtung;
- Fig. 2: eine Draufsicht auf das Ausführungsbeispiel der Fig. 1;
- Fig. 3: eine Draufsicht auf eine verdrehbare Fixiereinrichtung;

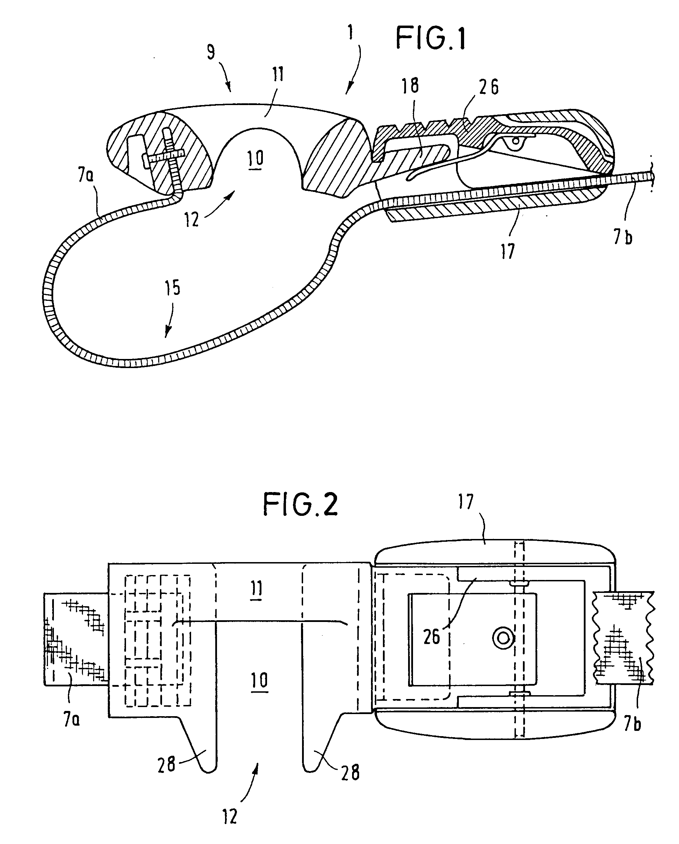

Figur 1 zeigt eine einstückige Fixiereinrichtung 9, die auf einer Seite das eine Ende 7a des Bandes 7 aufnimmt und die an dem anderen Ende ein Verschlußteil 18 aufweist, das in dem Schloßteil 17 einrastbar ist und dort mit einem in dem Schloßteil 17 schwenkbar gelagerten Klemmhebel 26 gekoppelt ist, der das durch das Schloßgehäuse hindurchgeführte Bandende 7b einerseits im gespannten Zustand festklemmt und andererseits durch Betätigung des Klemmhebels 26 auf der der Fixiereinrichtung 9 zugewandten Seite ein Lösen des Bandes 7 ermöglicht.

Die einstückige Fixiereinrichtung 9 kann dabei einen wie in den Figuren 1 und 2 dargestellt offenen Aufnahmekanal 12 aufweisen, der den Blutdurchfluß durch die in dem Spalt 10 eingefaßte Vene nicht unterbricht oder einen einseitig geschlossenen Aufnahmekanal 12 haben, der dadurch gebildet ist, daß der den Aufnahmekanal 12 überbrückende Steg 11 bis zur körperseitigen Unterkante der Fixiereinrichtung 9 verläuft. Die Fixiereinrichtung 9 kann zwei einseitig des Bandes 7 vorstehende Vorsprünge 28 aufweisen, die unmittelbar auf dem Körperteil anliegen und zwischen sich den Spalt 10 des Aufnahmekanals 12 freilassen.

Dieses Ausführungsbeispiel kann auch einen in der Ebene des Bandes 7 schwenkbaren Aufnahmekanal 12 aufweisen.

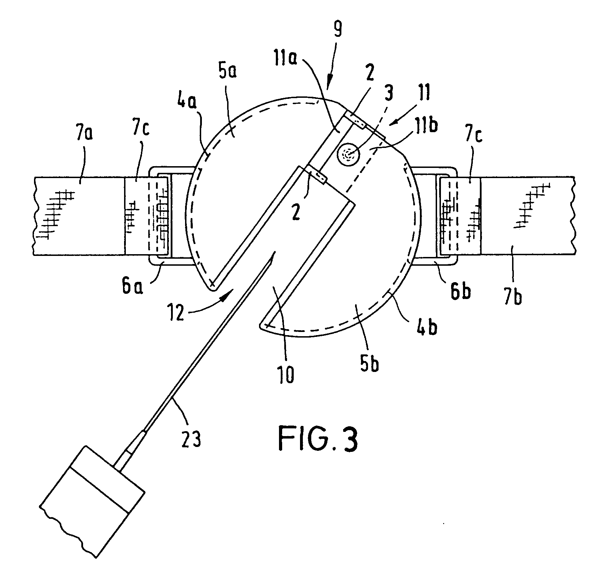

Zur Erläuterung der Schwenkbarkeit des Aufnahmekanals 12 wird auf Fig. 3 Bezug genommen, in der eine drehbare Fixiereinrichtung 9 einer Abschnürvorrichtung 1 in etwas vergrößertem Maßstab dargestellt ist. In dem Ausführungsbeispiel ist die Fixiereinrichtung 9 im Umriß überwiegend kreisrund gestaltet und besteht aus zwei etwa spiegelsymmetrischen Teilplatten 5a, 5b, die im Stegbereich 11 der Fixiereinrichtung zusammengefügt sind. Die Fixiereinrichtung 9 weist einen im Querschnitt etwa rechteckigen oder U-förmigen Spalt 10 auf, der sich radial erstreckt und zum einen Rand der Fixiereinrichtung hin nach außen offen ist und der sich in der Länge in etwa über zwei Drittel des Durchmessers der Fixiereinrichtung erstreckt. Die Breite des Spalts 10 ist dadurch variabel, daß die beiden Teilplatten 5a, 5b im Bereich des Stegs 11 gegeneinander verschiebbar sind, wobei eine Feststellschraube 3 vorgesehen ist, mittels derer die beiden Teilplatten in der jeweiligen Position mit der gewünschten Spaltbreite, die sich nach der Breite der Vene richtet, fixierbar sind. Auf der linken bzw. rechten Seite der Fixiereinrichtung 9 in Fig. 3 sind am Umfang der Fixiereinrichtung 9 jeweils die Enden 7a, 7b eines Abschnürmittels in Form eines Bandes an Befestigungsösen 6 befestigt.

Damit die Fixiereinrichtung 9 in eine gewünschte Lage bringbar ist, in der die zu punktierende Vene in dem Spalt 10 liegt, sind an den Teilplatten 5a, 5b am Umfang umlaufend Gleitschienen 4a, 4b in Form von hinterschnittenen Nuten ausgebildet, die zur Stirnseite der Teilplatten 5a, 5b hin offen sind, so daß stirnseitig ein durchgehender Spalt in den Teilplatten gebildet ist (s. Fig. 3). Die Befestigungsösen 6a, 6b, an denen die Schlaufen 7c der Bandenden 7a, 7b befestigt werden, weisen an der der Fixiereinrichtung zugewandten Seite jeweils Anformungen auf, die von den Gleitschienen 4a, 4b aufgenommen werden. Auf diese Weise sind die Befestigungsösen 6a, 6b in diesen Gleitschienen 4a, 4b entlang einer kreislinienförmigen Führungsbahn verschiebbar. Es ist ausreichend, wenn sich die Gleitschienen 4a, 4b jeweils über einen Teilbereich des Umfangs der Teilplatten 5a, 5b erstrecken. Die Gleitschienen 4a, 4b können beispielsweise jeweils mit etwas Abstand vor dem Spalt 10 enden und sich über den Krümmungsbereich der Teilplatten 5a, 5b bis etwa in Nähe des geradlinigen Stegbereichs 11 erstrecken. Die Fixiereinrichtung 9 kann somit gegenüber den Bandenden 7a, 7b um ihre Achse verdreht werden, bis die zu punktierende Vene in dem Spalt 10 liegt und in Spaltrichtung verläuft. Eine derartige Position mit verdrehter Fixiereinrichtung ist in Fig. 3 dargestellt. Die gestaute bzw. aufgeblähte Vene kann nun mit nun mittels der Injektionsnadel 23 punktiert werden.

Die vorzugsweise vorgesehene Möglichkeit, die beiden Teilplatten 5a, 5b gegeneinander zu verschieben und damit die Breite des Spalts 10 zu verändern, wird aus den Figuren 1 und 2 deutlich. Die untere Teilplatte 5a ist im Stegteil 11a an den äußeren Rändern um etwa 180° umgebogen ist, so daß sich an den Rändern eine Art Führungsschiene 2 ergibt, die den Stegteil 11b der Teilplatte 5b aufnimmt. Die Ränder der Teilplatte 5a umgreifen im Bereich des Stegs 11 den Rand der Teilplatte 5b, die sich im Stegbereich über die Teilplatte 5a schiebt und bei dieser Verschiebbewegung in der an der Teilplatte 5a gebildeten Führungsschiene 2 geführt wird. Der Spalt 10 kann beispielsweise auf eine Breite von ca. 8 mm im Normalfall eingestellt und je nach Größe der zu punktierenden Vene verstellt werden. Ist die gewünschte Spaltbreite eingestellt, werden die beiden Teilplatten 5a, 5b mittels der Feststellschraube 3 im Stegbereich fixiert. Die Flanken 8 des den Spalt 10 freihaltenden Aufnahmekanals 12 können schräg verlaufen, so daß der Spalt 10 im Querschnitt gesehen sich zum Körperteil konisch verengt.

## Patentansprüche

1. Abschnürvorrichtung für Körperteile mit einem ein Körperteil umfassendes, eine Schlinge (15) bildendes Abschnürmittel (7,7a,7b) mit einem Schloßteil (17), das das Abschnürmittel (7,7a,7b) in gespanntem Zustand arretieren und danach wieder entspannen kann, wobei in dem Schloßteil (17) ein Klemmhebel (26) verschwenkbar gelagert ist, der das durch das Schloßgehäuse hindurchgeführte Bandende (7b) einerseits im gespannten Zustand festklemmt und andererseits durch Betätigung des Klemmhebels (26) ein Lösen des Bandes (7) ermöglicht,
**dadurch gekennzeichnet,**
- daß das Abschnürmittel (7,7a,7b) eine Fixiereinrichtung (9) mit einem einen Spalt (10) bildenden Aufnahmekanal (12) für eine Vene enthält, der beim Abschnüren des Körperteils die Vene einfaßt und in dem Aufnahmekanal (12) fixiert,
- daß der Aufnahmekanal (12) durchgehend ist,
- daß die Fixiereinrichtung (9) im Bereich des Aufnahmekanals (12) einen nach oben hin aufgewölbten Steg (11,40) aufweist, so daß bei angelegter Fixiereinrichtung (9) unter dem Steg (11,40) ein tunnelartiger Freiraum verbleibt, der einen verstärkten Blutrückfluß durch die fixierte Vene zuläßt, während die Venen in der Umgebung der fixierten Vene gestaut werden, und
- daß die Fixiereinrichtung (9) auf einer Seite das eine Ende (7a) des Abschnürmittels (7) aufnimmt und auf der dem Ende (7a) gegenüberliegenden Seite ein Verschlußteil (18) aufweist, das mit dem in dem Schloßteil (17) verschwenkbar gelagerten Klemmhebel (26) lösbar gekoppelt ist.

2. Abschnürvorrichtung nach Anspruch 1, dadurch gekennzeichnet, daß die Fixiereinrichtung (9) einen relativ zum Abschnürmittel (7, 7a,7b) in der Ebene des Abschnürmittels schwenkbaren Aufnahmekanal (12) aufweist.

3. Abschnürvorrichtung nach einem der Ansprüche 1 oder 2, dadurch gekennzeichnet, daß die Fixiereinrichtung (9) im Umfangsbereich Gleitschienen (4a,4b) aufweist, in denen Anformungen von Befestigungsösen (6) auf einer Kreisbahn verschiebbar geführt sind.

4. Abschnürvorrichtung nach einem der Ansprüche 1 bis 3, dadurch gekennzeichnet, daß der Spalt (10) der Fixiereinrichtung (9) in seiner Breite einstellbar ist.

5. Abschnürvorrichtung nach einem der Ansprüche 1 bis 4, dadurch gekennzeichnet, daß die Fixiereinrichtung (9) aus zwei Teilplatten (5a,5b,48a,48b) besteht, die im Bereich des Stegs (11,40,44) zusammengefügt sind und gegeneinander verschiebbar sind, so daß sich die Spaltbreite verändert.

6. Abschnürvorrichtung nach Anspruch 5, dadurch gekennzeichnet, daß die beiden Teilplatten (5a,5b) im Bereich des Stegs (11) übereinanderschiebbar sind, wobei ein an einer Teilplatte (5b) angeformter Stegteil (11b) in einer schienenartigen Führungseinrichtung (2), die am Stegteil (11a) der anderen Teilplatte (5a) angeformt ist, geführt wird.

7. Abschnürvorrichtung nach einem der Ansprüche 5 oder 6, dadurch gekennzeichnet, daß eine Feststellschraube (3) zur Fixierung der beiden Teilplatten (5a,5b) im Stegbereich in der jeweiligen Verschiebeposition vorgesehen ist.

8. Abschnürvorrichtung nach einem der Ansprüche 1 bis 7, dadurch gekennzeichnet, daß der Aufnahmekanal (12) mit Schutzlaschen gegen Kontamination geschützt ist.

## Claims

1. A tourniquet device for extremities, comprising a constriction means (7,7a,7b), to be laid around an extremity, which constriction means (7,7a,7b) forms a loop (15) and is provided with a locking member (17) adapted to lock the constriction means (7,7a,7b) in the stretched condition and then release it again, the locking member (17) having a clamping lever (26) pivotably supported therein by which, on the one hand, the strap end (7b) guided through the lock housing can be clamped in the stretched condition and, on the other hand, the strap (7) can be released by actuating the clamping lever (26),
**characterized in**
- that the constriction means (7,7a,7b) comprises a fixing means (9) having a receiving channel (12) for a vein formed therein, said receiving channel (12) forming a gap (10) and, when constricting the extremity, enclosing the vein and fixing it in the receiving channel (12),
- that the receiving channel (12) is continuous,
- that the fixing means (9) in the region of the receiving channel (12) comprises an upwardly bulging web (11,40) such that, when the fixing means (9) has been applied, a tunnel-shaped free space remains under the web (11,40) to allow increased backflow of blood through the fixed vein while the veins surrounding the fixed vein are compressed, and
- that the fixing means (9) on one side accommodates one end (7a) of the constriction means (7) and on the side facing away from said end (7a) comprises a closure means (18) releasably coupled to the clamping lever (26) pivotably supported in the locking member (17).

2. The tourniquet device according to claim 1, characterized in that the fixing means (9) is provided with a receiving channel (12) pivotable relative to the constriction means (7,7a,7b) in the plane of the constriction means.

3. The tourniquet device according to claim 1 or 2, characterized in that the fixing means (9) in its peripheral region is provided with sliding rails (4a,4b) for guiding protrusions of fastening eyelets (6) along a circular path.

4. The tourniquet device according to any one of claims 1 to 3, characterized in that the width of the gap (10) of the fixing means (9) is adjustable.

5. The tourniquet device according to any one of claims 1 to 4, characterized in that the fixing means (9) consists of two partial plates (5a,5b, 48a,48b) connected in the region of the web (11, 40,44) and being displaceable relative to each other to change the width of said gap.

6. The tourniquet device according to claim 5, characterized in that the two partial plates (5a,5b) can be shifted above each other in the region of the web (11), and that a web portion (11b) formed on one partial plate (5b) is guided in a rail-like guide means (2) formed on the web portion (11a) of the other partial plate (5a).

7. The tourniquet device according to claim 5 or 6, characterized in that a fixing screw (3) for fixing the two partial plates (5a,5b) is provided in the web region in the respective displacement position.

8. The tourniquet device according to any one of claims 1 to 7, characterized in that the receiving channel (12) is provided with protective flaps against contamination.

## Revendications

1. Dispositif de ligature pour parties du corps, avec un moyen de ligature (7, 7a, 7b) formant une boucle (15) et entourant une partie du corps, avec un élément de fermeture (17) qui peut arrêter le moyen de ligature (7, 7a, 7b) à l'état tendu et ensuite à nouveau le détendre, dans l'élément de fermeture (17) étant monté, en rotation, un levier de serrage (26) qui, d'une part, fixe à l'état tendu l'extrémité de bande (7b) passée à travers le boîtier de serrure et, d'autre part, permet, en actionnant le levier de serrage (26), de desserrer la bande (7),
caractérisé par le fait
- que le moyen de ligature (7, 7a, 7b) comporte un arrangement de fixation (9) avec un canal de réception (12) de veine, formant une ouverture (10), qui, lors de la ligature de la partie du corps, encadre la veine et la fixe dans le canal de réception (12),
- que le canal de réception (12) traverse l'arrangement de fixation,
- que l'arrangement de fixation (9) présente, au niveau du canal de réception (12), un pont (11, 40) voûté vers le haut, de sorte qu'il reste sous le pont (11, 40), après la pose de l'arrangement de fixation (9), un espace libre en forme de tunnel qui permet un retour de sang accru à travers la veine fixée, tandis que les veines dans le voisinage de la veine fixée sont bloquées,
- que l'arrangement de fixation (9) reçoit, d'un côté, l'une (7a) des extrémités du moyen de ligature (7) et présente, du côté opposé à l'extrémité (7a), un élément de fermeture (18) qui est couplé, de manière amovible, avec le levier de serrage (26) monté de manière à pivoter dans l'élément de fermeture (17).

2. Dispositif de ligature selon la revendication 1, caractérisé par le fait que l'arrangement de fixation (9) présente un canal de réception (12) susceptible de pivoter par rapport au moyen de ligature (7, 7a, 7b), dans le plan du moyen de ligature.

3. Dispositif de ligature selon l'une des revendications 1 ou 2, caractérisé par le fait que l'arrangement de fixation (9) présente, dans la zone périphérique, des rails de glissement (4a, 4b) sur lesquels des bases de boucles de fixation (6) sont guidées, de manière mobile, sur un chemin circulaire.

4. Dispositif de ligature selon l'une des revendications 1 à 3, caractérisé par le fait que la largeur de l'ouverture (10) de l'arrangement de fixation (9) est réglable.

5. Dispositif de ligature selon l'une des revendications 1 à 4, caractérisé par le fait que l'arrangement de fixation (9) se compose de deux plaques partielles (5a, 5b, 48a, 48b) qui sont assemblées au niveau du pont (11, 40, 44) et peuvent se déplacer l'une par rapport à l'autre, de sorte que la largeur de l'ouverture change.

6. Dispositif de ligature selon la revendication 5, caractérisé par le fait que les deux plaques partielles (5a, 5b) peuvent glisser l'une sur l'autre au niveau du pont (11), un élément du pont (11b) formé sur une plaque partielle (5b) étant guidé dans un élément de guidage (2) en forme de rail qui est formé sur l'élément du pont (11a) de l'autre plaque partielle (5a).

7. Dispositif de ligature selon l'une des revendications 5 ou 6, caractérisé par le fait qu'il est prévu une vis de blocage (3) pour la fixation des deux plaques partielles (5a, 5b), au niveau du pont, dans chaque position de déplacement.

8. Dispositif de ligature selon l'une des revendications 1 à 7, caractérisé par le fait que le canal de réception (12) est protégé contre la contamination par des languettes de protection.
